Europäisches Patentamt

European Patent Office

Office européen des brevets

(19)

(11) Veröffentlichungsnummer: **0 261 242**
A1

(12) # EUROPÄISCHE PATENTANMELDUNG
Veröffentlicht nach Art. 158 Abs. 3 EPÜ

(21) Anmeldenummer: 86904462.8

(22) Anmeldetag: 31.03.86

(51) Int. Cl.³: **A 61 F 9/00**

Daten der zugrundeliegenden internationalen Anmeldung:

(86) Internationale Anmeldenummer:
PCT/SU86/00028

(87) Internationale Veröffentlichungsnummer:
WO87/05799 (08.10.87 87/22)

(43) Veröffentlichungstag der Anmeldung:
30.03.88 Patentblatt 88/13

(84) Benannte Vertragsstaaten:
BE DE FR GB IT LU NL

(71) Anmelder: MOSKOVSKY NAUCHO-ISSLEDOVATELSKY
INSTITUT MIKROKHIRURGII GLAZA
Beskudnikovsky bulvar, 59a
Moscow, 127486(SU)

(72) Erfinder: FEDOROV, Svyatoslav Nikolaevich
ul. Dostoevskogo, 21-32
Moscow, 103030(SU)

(72) Erfinder: SOLOVIEV, Sergei Anatolievich
ul. Palekhskaya, 147-1-348
Moscow, 129347(SU)

(72) Erfinder: DEGTEV, Evgeny Ivanovich
Yaroslavskoe shosse, 14-34
Moscow, 129348(SU)

(72) Erfinder: IVASHINA, Albina Ivanovna
ul. Dubninskaya, 71-109
Moscow, 127591(SU)

(72) Erfinder: KARAVAEV, Alexandr Alexandrovich
Michurinsky pr., 22/3-17
Moscow, 117192(SU)

(74) Vertreter: Finck, Dieter et al,
Patentanwälte v. Füner, Ebbinghaus, Finck Mariahilfplatz
2 & 3
D-8000 München 90(DE)

(54) ANORDNUNG ZUR DURCHFÜHRUNG VON AUGENOPERATIONEN.

(57) Das Gerät zur Druchführung von ophthalmologischen Operationen enthält einen Schaftkörper (1), ein Schneidwerkzeug (9) mit seiner Festeinklemmung (10), das an dem federbelasteten Stab (7) befestigt ist, der im Inneren des Schaftkörpers (1) untergebracht ist und an seinem anderen Ende ein Mikrometergewinde (11) trägt. An dem Schaftkörper (1) ist eine Auflagehülse (14) vermittels des Mikrometergewinde (15) aufgeschraubt, während an dieser Auflagehülse (14) ein Einklinkhalter deren Stellungslage angeordnet ist, der mit der den Stab (7) verschiebenden Mutter zusammengekoppelt ist.

./...

FIG.1

# GERÄT ZUR DURCHFÜHRUNG VON OPHTHALMOLOGISCHEN OPERATIONEN

## Technisches Gebiet

Die Erfindung bezieht sich auf chirurgische Instrumente, insbesondere auf Geräte zur Durchführung von ophthalmologischen Operationen.

## Vorangehender Stand der Technik

Bekannt ist ein Gerät zur Durchführung von ophthalmologischen Operationen, das einen Schaftkörper vorsieht, der ein unter Ermöglichung dessen Verstellung in Längsrichtung untergebrachtes Schneideinstrument und eine Auflegestütze trägt (s. beispielsweise SU, A, IOI6 882, Kl. A 6I F 9/00, I982, Moskovsky nauchno-issledovatelsky institut mikrokhirurgii glaza).

Das betreffende Gerät ist nicht imstande, eine genughohe Qualität der Durchführung von Operationen zu bewirken, was darauf zurückzuführen ist, dass der Einschnitt an einer erforderlichen Idealkurve entlang nicht verlegt werden kann.

Bekannt ist noch ein Gerät zur Durchführung von ophthalmologischen Operationen, das ebenfalls einen Schaftkörper, eine Auflegestütze einschliesst, die an dem Schaftkörper angeordnet ist und mit der Oberfläche der Hornhaut des Auges während der Operation in Wechselwirkung tritt. Im Inneren des Schaftkörpers ist ein federbelasteter Stab untergebracht, an dessen einem Ende das Schneideinstrument mit der Festeinklemmung befestigt ist und an dem anderen Ende das Mikrometergewinde zur Verstellung des Stabes innerhalb des Schaftkörpers vermittels einer Mutter ausgefräst ist (s. beispielsweise SU, A, 959 779, Kl. A 6I F 9/00, I982, Ju.N.Shirokov, S.A.Soloviev, V.P.Osetzky, S.N.Fedorov und V.V.Durnev).

Dieses Gerät ermöglicht ebenfalls keine genughohe Genauigkeit der Aufstellung des Schneideinstrumentes auf die vorgeschriebene Einschnittstiefe zu erreichen, wodurch auch die Genauigkeit bei der Durchführung von Operation beeinträchtigt wird.

Darüber hinaus ist das betreffende Gerät im Gebrauch unbequem, wodurch die Operationsqualität herabgesetzt wird, sowie eine bestimmte Zeitdauer notwendig ist, um das Gerät zu der Operation vorzubereiten, was sich bei der Verminderung dessen Gebrauchswirksamkeit auswirkt.

Offenbarung der Erfindung

Der Erfindung liegt die Aufgabe zugrunde, ein Gerät zur Durchführung von ophthalmologischen Operationen zu entwickeln, das eine hohe Genauigkeit bei der Aufstellung des Schneideinstrumentes auf die gewünschte Einschnittstiefe durch eine Abänderung der Ausführung der Aufstellungsmittel des Schneideinstrumentes erreichen lässt.

Die gestellte Aufgabe wird dadurch gelöst, dass das Gerät zur Durchführung von ophthalmologischen Operationen, das einen Schaftkörper, eine an dem Schaftkörper angeordnete und bei der Operation mit der Oberfläche der Hornhaut des Auges in Wechselwirkung tretende Auflegestütze, ein Schneideinstrument mit der Festeinklemmung, das an dem federbelasteten Stab befestigt ist, der innerhalb des Schaftkörpers untergebracht ist und an seinem anderen Ende ein Mikrometergewinde trägt, vermittels dessen das Schneideinstrument innerhalb des Schaftkörpers verstellt wird, und eine Mutter vorsieht, die mit dem Stab zu dessen Verstellung in Wechselwirkung tritt, erfindungsgemäss eine Auflagehülse, die am Schaftkörper vermittels des Mikrometergewindes auf dessen dem die Auflegestütze tragenden Schaftende gegenüberliegender Seite angeordnet ist, und einen Einklinkhalter der Stellungslage der Auflagehülse in Bezug auf den Schaftkörper trägt, der auf der Auflagehülse angeordnet ist und mit der Mutter in Wechselwirkung tritt.

Durch die erwähnte aufbaumässige Ausführung wird eine genauere Aufstellung der Auflegestütze und demzufolge auch des Schneidewerkzeugs in bezug auf die Hornhaut des Auges erreicht.

Zweckmässigerweise ist der Einklinkhalter der Stellungslage der Auflagehülse mit einem federbelasteten Einklinkhebel zu versehen, dessen Achse an der Aussenmantelfläche der Auflagehülse angelenkt ist und der an seinem einen Ende mit den Ausfräsungen im Schaftkörper und in der Auflagehülse in Wechselwirkung tritt, welche Ausfräsungen derart ausgeführt sind, dass die beiden bei der Festlegung der Stellungslage der Auflagehülse vereint werden, wobei die Ausfräsung in der Auflagehülse hindurchge-hend ausgeführt wird, während das andere Hebelende mit der Mutter vermittels auf diesen beiden aufgetragener Riffelungen zusammenwirkt.

Nicht minder zweckmässig ist das Mikrometergewinde auf dem Stab und auf der Auflagehülse mit unterschiedlicher Steigerung auszuführen.

Kurzbeschreibung der Zeichnungen

Nachstehend wird die Erfindung anhand der Beschreib-ung deren Ausführungsbeispiels unter Bezugnahme auf die begleitende Zeichnungen erläutert, in denen     zeigt:

Fig. I erfindungsgemässes Gerät zur Durchführung von ophthalmologischen Operationen im Längsschnitt;

Fig. 2 Ansicht in Richtung A auf die Skala der Mikrometerschraube desselben Geräts;

Fig. 3 Schnitt III-III desselben Geräts aus der Fig. I.

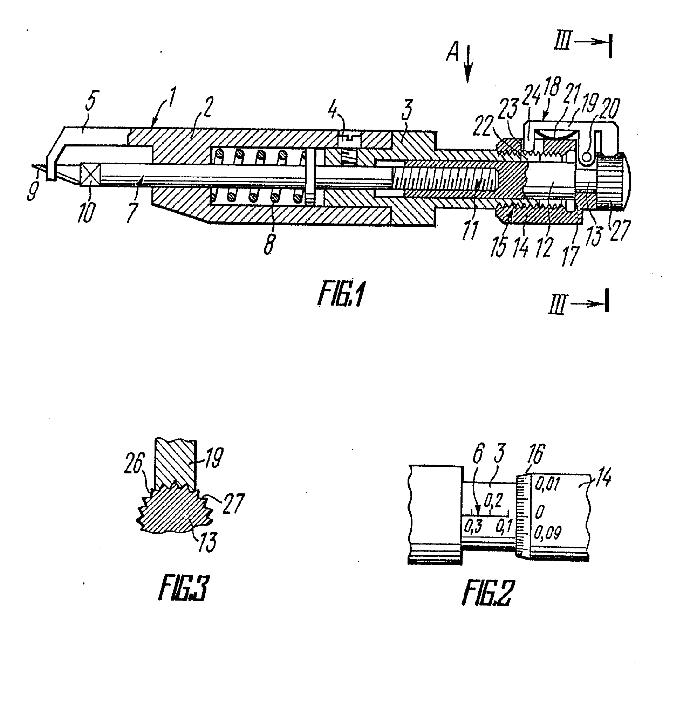

Beste Ausführungsvariante der Erfindung

Das erfindungsgemässe Gerät zur Durchführung von ophthalmologischen Operationen enthält einen hohlen Schaftkörper I (Fig. I), der aus zwei durch Schraube 4 zusammenverbundenen Schaftteilen 2 und 3 besteht. Der Schaftteil 2 des Schaftkörpers I trägt eine während der Operationen mit der Oberfläche der Hornhaut des Auges in Wechselwirkung tretende Auflegestütze 5, während auf dem Schaftteil 3 des Schaftkörpers I eine Grobskala 6 (Fig. 2) aufgetragen ist. Innerhalb des Schaftkörpers I (Fig. I) ist ein durch Feder 8 belasteter Stab 7 untergebracht, an dem das

-4-                                    0261242

Schneidwekzeug 9 vermittels dessen Festeinklemmung IO befestigt ist. An anderem Ende des Stabes 7 ist das Mikrometergewinde II der Steigerung $t_I$ aufgetragen, mit dessen Hilfe der Stab 7 mit der Mutter I3 über die Welle I2 im Laufe des Aufstellungsvorganges des Schneidwerkzeuges 9 in Zusammenwirkung tritt.

An dem Schaftteil 3 des Schaftkörpers I ist eine Auflagehülse I4 vermittels des Mikrometergewindes I5 aufgesetzt, das die Steigung $t_2$ aufweist, wobei $t_I \neq t_2$. Auf der Auflagehülse I4 ist eine Feinskala I6 (Fig. 2) der erhöhten Genauigkeit aufgetragen. Gegen die Innenstirnfläche I7 (Fig. I) der Auflagehülse I4 stösst die Stirnflanke der Welle I2. Auf der Auflagehülse I4 ist nocht der Einklinkhalter I8 deren Stellungslage in bezug auf den Schaftkörper I montiert.

Der Einklinkhalter I8 enthält einen Einklinkhebel I9, der auf der Achse 20 an der Aussenmantelfläche der Auflagehülse I4 angelenkt und durch die Feder 2I belastet ist.

In dem Schaftteil 3 des Schaftkörpers I ist eine Eindrehung 22 und in der Auflagehülse I4 einen durchgehenden Ausschnitt 23 ausgespart, die beiden mit dem Arm 24 des Einklinkhebels I9 in Wechselwirkung treten. An dem anderen Arm 25 des Einklinkhebels I9 sind Riffelungen 26 (Fig. 3) ausgefräst, die mit solchen Riffelungen 27 auf der Mutter I3 übereinstimmen.

Das erfindungsgemässe Gerät arbeitet wie folgt.

Zunächst wird das Schneidwerkzeug 9 in die Festeinklemmung IO eingesetzt. Beim Drücken mit dem Finger an dem Einklinkhebel I9 des Einklinkhalters I8 wird der Ausschnitt 23 der Auflagehülse I4 mit der Eindrehung 22 des Schaftkörpers I vereint. Dabei ragt der Einklinkarm 24 des Hebels I9 in den Ausschnitt 23 und in die Eindrehung 22 gleichzeitig hinein.

Diese Stellungslage der Auflagehülse I4 in bezug auf den Schaftkörper I fällt mit der Einstellung und Festlegung der Nullstellung auf der Grobskala 6 und der Nullstellung der Feinskala I6 zusammen. Bei dieser Stellungslage steht die Mutter I3 mit der Auflagehülse I4 ausser dem

Eingriff, da die Riffelungen 26 in die Riffelungen 27 der Mutter I3 nicht eingreifen. Hiernach wird das Schneidwerkzeug 9 unter dem Drehen an der Mutter I3 mit der Oberfläche der Auflagestütze 5 bündiggestellt. Im weiteren wird es an dem Einklinkhebel I9 mit dem Finger gedrückt, wodurch dieser durch die Feder 2I von der Mantelfläche der Auflegehülse I4 hinweggestossen wird und der Hebelarm 24 aus der Eindrehung 22 und dem Ausschnitt 23 entsprechenderweise des Schaftkörpers I und der Auflagehülse I4 heraustritt. In diesem Zusammenhang treten die Riffelungen 26 des Einklinkhebels I9 infolge dessen Anlenkung an der Auflagehülse I4 mit den Riffelungen 27 auf der Mutter I3 in Eingriff.

Hiernach wird das Schneidwerkzeug 9 an der Skala 6 des Schaftkörpers I und an der Skala I6 der Auflagehülse I4 durch Drehen an der Mutter I3 bzw. der Auflagehülse I4 auf die gewählte, einer notwendigen Einschnittstiefe entsprechende Grösse hervorgeschoben, wodurch das Gerät vollkommen gebrauchsbereit ist.

Da die Mutter I3 mit der Auflagehülse I4 zusammenwirkt und die Stirnflanke der Welle I2 gegen die Innenstirnfläche I7 der Auflagehülse I4 drückt, wird das Schneidwerkzeug 9 bei einer Umdrehung der Mutter I3 auf die Vorschubgrösse hervorgeschoben, die der Differenz der Steigungen des Gewindes der Mutter I3 und des Gewindes in der Auflagehülse I4 entspricht.

Die Anwendung des erfindungsgemässen Gerätes macht es möglich, eine die Genauigkeit der Durchführung der Operation etwa auf das 10fache zu steigern.

Diese Steigerung der Operationsgenauigkeit ist darauf zurückzuführen, dass der Aufbau des erfindungsgemässen Geräts den Vorschub des Schneidwerkzeugs je eine Umdrehung der Mutter auf eine nur geringe Grösse und eine genaue Einstellung des Geräts in dessen Nullstellungslage ermöglicht, was es gestattet, notwendigerfalls das im Laufe der Operation stumpfgewordene Schneidwerkzeug auszuwechseln.

0261242

## Industrielle Verwendbarkeit

Die vorliegende Erfindung kann zur Ausführung der in ihrer Tiefe und Gestalt vorgeschriebenen Blindeinschnitte der Hornhaut bei Operationen am Auge zwecks Veränderung dessen Refraktion ihre Anwendung finden.

PATENTANSPRUCHE

I. Gerät zur Durchführung von ophthalmologischen Operationen, das einen Schaftkörper (I), eine innerhalb des Schaftkörpers (I) untergebrachte und mit der Oberfläche der Hornhaut des Auges im Laufe der Operation in Wechselwirkung tretende Auflegestütze (5), ein Schneidwerkzeug (9) mit seiner Festeinklemmung (IO), das an einem federbelasteten Stab (7) befestigt ist, der im Inneren des Schaftkörpers (I) untergebracht ist und an seinem anderen Ende das Mikrometergewinde (II) trägt, mit dessen Hilfe er im Inneren des Schaftkörpers (I) verschoben wird, und eine Mutter (I3) vorsieht, die mit dem Stab (7) zu dessen Verschiebung zusammenwirkt, dadurch g e k e n n z e i c h - n e t, dass es mit einer Auflagehülse (I4) ausgestattet ist, die an dem Schaftkörper (I) vermittels des Mikrometergewindes (I⁵) auf dessen dem die Auslegestütze (5) tragenden Ende gegenüberliegender Seite aufgesetzt ist, und einen Einklinkhalter (I8) der Stellungslage der Auflagehülse (I4) in bezug auf den Schaftkörper (I) besitzt, der an der Auflagehülse (I4) angeordnet ist und mit der Mutter (I3) zusammenwirkt.

2. Gerät nach Anspruch I, dadurch g e k e n n - z e i c h n e t, dass der Einklinkhalter (I8) der Stellungslage der Auflagehülse (I4) einen federbelasteten Einklinkhebel (I9) enthält, dessen Achse (20) auf der Aussenmantelfläche der Auflagehülse (I4) angelenkt ist und der mit seinem einen Einklinkarm (24) mit der Eindrehung (22), und dem Ausschnitt (23) in dem Schaftkörper (I) und in der Auflagehülse (I4) in Eingriff tritt, die derart ausgeführt sind, dass diese zum Festlegen der Stellungslage der Auflagehülse (I4) vereint sind, wobei der Ausschnitt (23) in der Auflagehülse (I4) einen durchgehenden Ausschnitt darstellt.

3. Gerät nach Anspruch 2, dadurch g e k e n n - z e i c h n e t, dass der zweite Hebelarm (25) des Einklinkhebels (I9) mit der Mutter (I3) vermittels Riffelungen (26, 27) an diesen beiden in Eingriff tritt.

4. Gerät nach Anspuch I, 2 bzw. 3, dadurch g e - k e n n z e i c h n e t, dass in diesem das Mikrometergewinde (II) auf dem Stab (7) und das Mikrometergewinde (I5) in der Auflagehülse (I4) unterschiedliche Steigerungen aufweisen.

0261242

1/1

FIG.1

FIG.3

FIG.2

# INTERNATIONAL SEARCH REPORT

0261242

International Application No PCT/SU 86/00028

## I. CLASSIFICATION OF SUBJECT MATTER (if several classification symbols apply, Indicate all) *

According to International Patent Classification (IPC) or to both National Classification and IPC

$IPC^4$ - A 61 F 9/00

## II. FIELDS SEARCHED

### Minimum Documentation Searched [7]

| Classification System | Classification Symbols |
|---|---|
| $IPC^4$ | A 61 F 9/00, A 61 B 17/32 |

Documentation Searched other than Minimum Documentation
to the Extent that such Documents are Included in the Fields Searched [8]

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| A | DE, C2, 3300567, (Moskovskij naučno-issledova tel'skij institut mikrochirurgii glaza), 13 February 1986 (13.02.86) | 1 |
| A | US, A, 4473076, (VXTRA Development Limited 700 Division), 25 August 1984 (25.09.84) | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 29 October 1986 (29.10.86) | 02 December 1986 (02.12.86) |
| International Searching Authority | Signature of Authorized Officer |
| ISA/SU | |

Form PCT/ISA/210 (second sheet) (January 1985)